Europäisches Patentamt

European Patent Office

Office européen des brevets

(11) Veröffentlichungsnummer: **0 382 066**
**A2**

(12) **EUROPÄISCHE PATENTANMELDUNG**

(21) Anmeldenummer: **90101867.1**

(51) Int. Cl.⁵: **A61K 31/19**

(22) Anmeldetag: **31.01.90**

Die Bezeichnung der Erfindung wurde geändert (Richtlinien für die Prüfung im EPA, A-III, 7.3).

(30) Priorität: **09.02.89 DE 3903758**

(43) Veröffentlichungstag der Anmeldung:
**16.08.90 Patentblatt 90/33**

(84) Benannte Vertragsstaaten:
**AT BE CH DE DK ES FR GB GR IT LI LU NL SE**

(71) Anmelder: **ASTA Pharma AG**
**Weismüllerstrasse 45**
**D-6000 Frankfurt am Main 1(DE)**

(72) Erfinder: **Ulrich, Heinz, Dr.**
**Bayernstrasse 2**
**D-8751 Niedernberg(DE)**
Erfinder: **Weischer, Carl-Heinrich, Dr.**
**Schmidtbonnstrasse 8**
**D-5300 Bonn 1(DE)**
Erfinder: **Engel, Jürgen, Dr.**
**Erlenweg 3**
**D-8755 Alzenau(DE)**
Erfinder: **Hettche, Helmut, Dr.**
**Martinstrasse 23**
**D-6057 Dietzenbach(DE)**

(54) **Verwendung von Dihydroliponsäure als Analgetikum, Antiphlogistikum und/oder Zytoprotektivum.**

(57) Arzneimittel und deren Herstellung, die als Wirkstoff Dihydroliponsäure oder deren pharmazeutisch verwendbare Salze enthalten. Die Arzneimittel besitzen eine zytoprotektive Wirkung und eignen sich zur Bekämpfung von Schmerz- und Entzündungserkrankungen.

EP 0 382 066 A2

## Arzneimittel enthaltend als Wirkstoff Dihydroliponsäure

Dihydroliponsäure ist die 6, 8-Dimercapto-octansäure. Aus Tieruntersuchungen ist bekannt, daß Dihydroliponsäure Schlangengift inaktiviert. Diese Untersuchungen erfolgten beispielsweise an Ratten und Mäusen, wobei Lösungen in Wasser oder physiologische Kochsalzlösung verwendet wurden, die das Schlangengift und Dihydroliponsäure enthielten.

Aufgabe der Erfindung ist die Bereitstellung von verbesserten Arzneimitteln mit beispielsweise analgetischer, antiphlogistischer und zytoprotektiver Wirkung.

Die Erfindung betrifft Arzneimittel , die als Wirkstoff Dihydroliponsäure oder ein pharmazeutisch verwendbares Salz der Dihydroliponsäure enthalten, deren Herstellung sowie die Verwendung der Dihydroliponsäure oder deren Salze zur Herstellung von Arzneimitteln. Diese besitzen insbesondere eine zytoprotektive Wirkung und sind außerdem zur Bekämpfung von Schmerzzuständen und Entzündungserkrankungen geeignet.

Die in den Patentansprüchen angegebenen Gewichtsmengen beziehen sich jeweils auf die reine Dihydroliponsäure, das heißt nicht auf die Salze. Bei Verwendung von Salzen müssen die jeweils in Frage kommenden Dosismengen entsprechen und dem geänderten Molgewicht entsprechend erhöht werden.

Vorzugsweise wird die Dihydroliponsäure als freie Säure verwendet, in wässrigen Lösungen in Form des Salzes mit pharmazeutisch verwendbaren Salzbildnern.

Die Dihydroliponsäure kann als Racemat oder in Form der beiden optischen Isomeren verwendet werden (R( + )- und S(-)-Form).

Die Dihydroliponsäure zeigt beispielsweise im Essigsäure-Writhing-Schmerztest an der Maus und im Randall-Selitto-Entzündungsschmerztest an der Ratte eine gute analgetische Wirkung, die der $\alpha$-Liponsäure um mindestens einen Faktor 1.9 beziehungsweise 2.8 überlegen ist (perorale Applikation).

Weiterhin zeigt die Dihydroliponsäure beispielsweise im Carrageenin-Ödem an der Ratte eine gute antiphlogistische Wirkung, die der $\alpha$-Liponsäure ebenfalls um mindestens Faktor 1.9 überlegen ist (perorale Applikation). Ebenso zeigt die Dihydroliponsäure eine gute Hemmung der Vasopermeabilität beispielsweise im PAF-Ödem an der Ratte sowie eine gute zytoprotektive Wirkung.

So wird beispielsweise in dem vorstehend angegebenen Essigsäure-Writhing-Test eine analgetisch wirksame ED50 der Dihydroliponsäure von 26.8 mg/kg per os beziehungsweise 4.6 mg/kg intraperitoneal ermittelt.

In dem vorstehend angegebenen Randall-Selitto-Test ergibt sich eine analgetisch wirksame ED50 der Dihydroliponsäure zu 16.2 mg/kg per os beziehungsweise 0.14 mg/kg intraperitoneal.

In dem vorstehend angegebenen Carrageenin-Ödem-Test wird zum Beispiel eine antiphlogistisch wirksame ED50 der Dihydroliponsäure von 26 mg/kg per os beziehungsweise 7.7 mg/kg intraperitoneal ermittelt.

Die niedrigste, bereits analgetisch wirksame Dosis in dem Randall-Selitto-Schmerztest ist beispielsweise 5 mg/kg per os.

Die niedrigste, bereits antiphlogistisch wirksame Dosis in dem Carrageenin-Ödem-Test ist beispielweise 5 mg/kg per os.

Ebenso tritt die zytoprotektive Wirkung im Tierversuch bereits ab einer Dosis von 5 mg/kg per os ein.

Als allgemeiner Dosisbereich für die analgetische Wirkung kommt beispielsweise in Frage:

5 - 100 mg/kg oral

Als allgemeiner Dosisbereich für die antiphlogistische und zytoprotektive Wirkung kommt beispielweise in Frage:

5 - 100 mg/kg oral

Die Wirkung der Dihydroliponsäure geht auch aus der folgenden Tabelle hervor:

| Versuchsmodell | ED 50 mg/kg | % Wirkung |
|---|---|---|
| Carrageenin-ödem | 26 per os<br>7,7 intraperitoneal | |
| Randall-Selitto-Entzündungsschmerz | 16,2 per os<br>0,14 intraperitoneal | |
| Essigsäure-Writhing-Test | 26,8 per os<br>4,6 intraperitoneal | |
| PAF-Acether-ödem | 25 mg/kg intraperitoneal<br>50 mg/kg intraperitoneal<br>200 mg/kg intraperitoneal | 14 %<br>63 %<br>73 % |

Die Dihydroliponsäure zeigt weiterhin an folgenden Untersuchungsmodellen eine gute analgetische, antiphlogistische, antiarthrotische und zytoprotektive Wirkung:

$MgSO_4$-Writhing-Test an der Maus nach GYIRES et al. (Arch.int.pharmacodyn.therap. 267, 131-140, 1984)

Adjuvans-Arthritis an der Ratte nach NEWBOULD (Brit.J.Pharmacol. 21, 127-136, 1963)

TPA- beziehungsweise Arachidonsäure-induziertes Mäuseohrödem nach YOUNG et al. (J.Invest.Dermatol. 80, 48-52, 1983)

Na-Monojodazetat-induzierte Arthrose an Ratten beziehungsweise Hühnern nach KALBHEN, in: Arthrosis deformans, Eular-Verlag, Basel/Schweiz, 1982

TPA-induzierte Arthrose an Ratten nach WEISCHER (Agents and Actions 23, 1/2, 1988)

Intestinale Ulzeration an Ratten nach DEL SOLDATO (Agents and Actions 16, 393-396, 1985)

Colitis-Modell an der Ratte nach WEISCHER (Agents and Actions, 1988)

Ethanol-Ulkus-Modell an der Ratte (Nachweis für beispielsweise eine zytoprotektive Wirkung)


Wirkung von topischer DL-Dihydroliponsäure bei dem durch UV-B-Bestrahlung induzierten Symptomenkomplex an gesunden Probanden


Die Dihydroliponsäure hemmt beispielsweise die akute Entzündung und den Entzündungsschmerz. Zum Beispiel ist sie auch geeignet zur Behandlung von Entzündungen der Gefäße und des Gefäßbindegewebes, wie zum Beispiel bei Arteriosklerose etc..

Dihydroliponsäure besitzt eine spezifische zytoprotektive Wirkung.

Als Indikationen kommen zum Beispiel in Betracht:

Entzündliche, degenerative artikuläre und extraartikuläre rheumatische Erkrankungen, nichtrheumatische Entzündungs- und Schwellungszustände, Arthrosis deformans, Chondropathien, Periarthritiden, entzündliche und nichtentzündliche Erkrankungen der Haut, wie z. B. Neurodermitis und Psoriasis, entzündliche und nichtentzündliche Erkrankungen des Gastrointestinaltraktes, wie zum Beispiel Gastritis, Ulcus ventriculi, Ileitis, Duodenitis, Jejunitis, Colitis, Polyneuropathie diabetogener, alkoholischer hepatischer und urämischer Genese, Leberparenchymdegeneration, Hepatitis, Fettleber und Fettzirrhose sowie chronische Lebererkrankungen, entzündliche Atemwegserkrankungen, zum Beispiel Asthma bronchiale, Sarkoidose, ARDS (acute respiratory distress syndrome).

Die Tagesdosen der erfindungsgemäßen Darreichungsformen für die analgetische, zytoprotektive und antiphlogistische Wirkung bestehen zum Beispiel aus 0,1 bis 600 mg, vorzugsweise 15 bis 400 mg und insbesondere 50 bis 200mg Dihydroliponsäure.

Die Wirkungsrichtung der Dihydroliponsäure ist mit der Wirkung der bekannten Wirkstoffe α-Liponsäure und S-Adenosyl-L-Methionin vergleichbar, jedoch bestehen hierzu insbesondere folgende Unterschiede:

1. Die volle Wirkung tritt im Tierversuch schon 0,5 Stunden nach Applikation ein und nicht wie bei S-Adenosyl-L-Methionin nach mehrmaliger Gabe.

2. Die Wirkung der Dihydroliponsäure ist beispielsweise um Faktor 2 - 3 stärker als die der α-Liponsäure.

Erfindungsgemäß wird eine Tagesdosis der Dihydroliponsäure von 10 - 600 mg, beispielsweise von 25 bis 400 mg oder 10 bis 200 mg verabreicht. Die maximale Tagesdosis soll 600 mg nicht überschreiten. Die Tagesdosen können in Form einer einmaligen Verabreichung der gesamten Menge oder in Form von 1 bis 6, insbesondere 1 bis 4, Teildosen pro Tag eingesetzt werden. Im allgemeinen ist eine Verabreichung von 1- bis 4mal, insbesondere 1- bis 3mal täglich bevorzugt. Beispielsweise beträgt die bevorzugte Tagesdosis für die Dihydroliponsäure vorzugsweise 80 mg für die parenterale Applikationsform und 200 mg für die orale Form. Insbesondere beträgt die Tagesdosis für die parenterale Applikationsform 50 mg und 150 mg für die orale Form.

Vorzugsweise wird das Arzneimittel oral verabreicht.

Die Dihydroliponsäure kann insbesondere auch in Form einer Lösung appliziert werden, beispielsweise peroral, topisch, parenteral (intravenös, intraartikulär, intramuskulär, subkutan), inhalativ, rektal, transdermal oder vaginal.

Die Arzneimittel, die als Wirkstoff die Dihydroliponsäure enthalten, können zum Beispiel in Form von Tabletten, Kapseln, Pillen oder Dragees, Granulaten, Suppositorien, Pellets, Pflaster, Lösungen oder Emulsionen formuliert werden, wobei der Wirkstoff mit entsprechenden Hilfs- und Trägerstoffen kombiniert wird. Im Falle von Lösungen enthalten diese beispielsweise 0,5 bis 20 Gewichts%, vorzugsweise 1 bis 10 Gewichts% Dihydroliponsäure.

Die Dosierungseinheit der Arzneimittel mit der Dihydroliponsäure oder einem therapeutisch verwendbaren Salz derselben kann beispielsweise enthalten:

a) bei peroralen Arzneiformen:

10 bis 600 mg, vorzugsweise 20 bis 400 mg, insbesondere 50 bis 200 mg Dihydroliponsäure.

Die Dosen können beispielweise 1- bis 6-, vorzugsweise 1- bis 4-, insbesondere 1- bis 3mal täglich verabreicht werden. Jedoch soll eine Gesamtdosis von 600 mg pro Tag nicht überschritten werden. Dasselbe gilt auch für die folgenden unter b) bis e) aufgeführten Arzneiformen.

b) bei parenteralen Arzneiformen (zum Beispiel intravenös, intramuskulär oder intraartikulär):

10 bis 300 mg, vorzugsweise 15 bis 200 mg, insbesondere 20 bis 100 mg Dihydroliponsäure.

Die Dosen können beispielsweise 1- bis 6-, vorzugsweise 1- bis 4-, insbesondere 1- bis 3mal täglich verabreicht werden.

c) bei Arzneiformen zur rektalen oder vaginalen Applikation:

10 bis 500 mg, vorzugsweise 40 bis 400, insbesondere 50 bis 200 mg Dihydroliponsäure.

Diese Dosen können beispielsweise 1- bis 6-, vorzugsweise 1- bis 4-, insbesondere 1- bis 3mal täglich verabreicht werden.

d) bei Arzneiformen zur Applikation auf die Haut und Schleimhäute (zum Beispiel als Lösungen, Lotionen, Emulsionen, Salben, Pflaster und so weiter):

10 bis 500 mg Dihydroliponsäure, vorzugsweise 40 bis 250 mg, insbesondere 50 bis 200 mg Dihydroliponsäure.

Die Dosen können beispielsweise 1- bis 6-, vorzugsweise 1- bis 4-, insbesondere 1- bis 3mal täglich verabreicht werden.

e) Bei Arzneiformen zur Inhalation (Lösungen oder Aerosole):

0,1 bis 300 mg, vorzugsweise 0,25 bis 150 mg, insbesondere 0,5 bis 80 mg Dihydroliponsäure.

Diese Dosen können beispielsweise 1- bis 6-, vorzugsweise 1- bis 4-, insbesondere 1- bis 3mal täglich verabreicht werden.

Falls Lösungen verwendet werden, wird die Dihydroliponsäure vorzugsweise in Form eines Salzes eingesetzt.

Selbstverständlich können auch galenische Zübereitungen hergestellt werden, welche die oben angegebenen Dosierungseinheiten 2- bis beispielsweise 6mal enthalten. Insbesondere enthalten Tabletten oder Kapseln 20 bis 500 mg, Pellets, Pulver oder Granulate 20 bis 400 mg, Suppositorien 20 bis 300 mg Dihydroliponsäure.

Die akute Toxizität der Erfindung an der Maus (ausgedrückt als LD50 mg/kg; Methode LITCHFIELD und WILCOXON, J. Pharmacol. Exp. Ther. 95:99, (1949)) liegt beispielsweise oberhalb von 200 mg/kg bei oraler Applikation.

Die vorstehend angegebenen Dosierungen beziehen sich stets auf die freie Dihydroliponsäure. Falls die Dihydroliponsäure in Form eines Salzes verwendet wird, sind die angegebenen Dosierungen/Dosierungsbereiche infolge des höheren Mol-Gewichts entsprechend zu erhöhen.

Für den Fall, daß die Dihydroliponsäure bei Tieren eingesetzt wird, kommen insbesondere folgende Indikationen in Frage: Hepatosen, Athrosis deformans, Arthritiden und Dermatitiden.

Beispiele für die Behandlung von Tieren:

Für die Behandlung von Hunden und Katzen liegt die orale Einzeldosis im allgemeinen zwischen ungefähr 2 mg/kg und 50 mg/kg Körpergewicht, die parenterale Dosis ungefähr zwischen 0,5 und 40 mg/kg Körpergewicht.

Für die Behandlung von Arthrosen bei Pferden und Vieh liegt die orale Einzeldosis im allgemeinen zwischen ungefähr 2 mg/kg und 100 mg/kg Körpergewicht, die parenterale Dosis ungefähr zwischen 0,5 und 50 mg/kg Körpergewicht.

Die Dihydroliponsäure ist zur Herstellung pharmazeutischer Zusammensetzungen und Zübereitungen geeignet. Die pharmazeutischen Zusammensetzungen beziehungsweise Arzneimittel enthalten die Dihydroliponsäure als Wirkstoff, gegebenenfalls in Mischung mit anderen pharmakologisch beziehungsweise pharmazeutisch wirksamen Stoffen. Die Herstellung der Arzneimittel erfolgt in bekannter Weise, wobei die bekannten und üblichen pharmazeutischen Hilfsstoffe sowie sonstige übliche Träger- und Verdünnungsmittel verwendet werden können. Als derartige Träger- und Hilfsstoffe kommen zum Beispiel solche Stoffe in Frage, die in folgenden Literaturstellen als Hilfsstoffe für Pharmazie, Kosmetik und angrenzende Gebiete empfohlen beziehungsweise angegeben sind: Ullmanns Encyklopädie der technischen Chemie, Band 4 (1953), Seite 1 bis 39; Journal of Pharmaceutical Sciences, Band 52 (1963), Seite 918 und ff., H. v. Czetsch-Lindenwald, Hilfsstoffe für Pharmazie und angrenzende Gebiete; Pharm. Ind., Heft 2, 1961, Seite 72 und ff.;

Dr. H. P. Fiedler, Lexikon der Hilfsstoffe für Pharmazie, Kosmetik und angrenzende Gebiete Cantor KG. Aulendorf in Württemberg 1981.

Beispiele hierfür sind Gelatine, natürliche Zucker wie Rohrzucker oder Milchzucker, Lecithin, Pektin, Stärke (zum Beispiel Maisstärke oder Amylose), Cyclodextrine und Cyclodextrinderivate, Dextran, Polyvinylpyrrolidon, Polyvinylacetat,

Gummi arabicum, Alginsäure, Tylose, Talkum, Lycopodium, Kieselsäure (zum Beispiel kolloidale), Cellulose, Cellulosederivate (zum Beispiel Celluloseether, bei denen die Cellulose-Hydroxygruppen teilweise mit niederen gesättigten aliphatischen Alkoholen und/oder niederen gesättigten aliphatischen Oxyalkoholen verethert sind, zum Beispiel Methyloxypropylcellulose, Methylcellulose, Hydroxypropylmethylcellulose, Hydroxypropylmethylcellulosephthalat); Fettsäuren sowie Magnesium-, Calcium- oder Aluminiumsalze von Fettsäuren mit 12 bis 22 C-Atomen, insbesondere der gesättigten (zum Beispiel Stearate), Emulgatoren, Öle und Fette, insbesondere pflanzliche (zum Beispiel Erdnußöl, Rizinusöl, Olivenöl, Sesamöl, Baumwollsaatöl, Maisöl, Weizenkeimöl, Sonnenblumensamenöl, Kabeljau-Leberöl, jeweils auch hydriert); Glycerinester und Polyglycerinester aus gesättigten Fettsäuren $C_{12}H_{24}O_2$ bis $C_{18}H_{36}O_2$ und deren Gemische, wobei die Glycerin-Hydroxygruppen vollständig oder auch nur teilweise verestert sind (zum Beispiel Mono-, Di- und Triglyceride); pharmazeutisch verträgliche ein- oder mehrwertige Alkohole und Polyglykole wie Polyethylenglykole sowie Derivate hiervon, Ester von aliphatischen gesättigten oder ungesättigten Fettsäuren (2 bis 22 C-Atome, insbesondere 10 - 18 C-Atome) mit einwertigen aliphatischen Alkoholen (1 bis 20 C-Atome) oder mehrwertigen Alkoholen wie Glykolen, Glycerin, Diethylenglykol, Pentaerythrit, Sorbit, Mannit und so weiter, die gegebenenfalls auch verethert sein können, Ester der Zitronensäure mit primären Alkoholen, Essigsäure, Harnstoff, Benzylbenzoat, Dioxolane, Glyzerinformale, Tetrahydrofurfurylalkohol, Polyglykolether mit $C_1$-$C_{12}$-Alkoholen, Dimethylacetamid, Lactamide, Lactate, Ethylcarbonate, Silicone (insbesondere mittelviskose Polydimethylsiloxane), Calciumcarbonat, Natriumcarbonat, Calciumphosphat, Natriumphosphat, Magnesiumcarbonat und ähnliche.

Als weitere Hilfsstoffe kommen auch Stoffe in Frage, die den Zerfall bewirken (sogenannte Sprengmittel) wie: quervernetztes Polyvinylpyrrolidon, Natriumcarboxymethylstärke, Natriumcarboxymethylcellulose oder mikrokristalline Cellulose. Ebenfalls können bekannte Hüllstoffe verwendet werden. Als solche kommen beispielsweise in Frage: Polymerisate sowie Copolymerisate der Acrylsäure und/oder Methacrylsäure und/oder deren Ester; Copolymerisate aus Acryl- und Methacrylsäureestern mit einem geringen Gehalt an Ammoniumgruppen (zum Beispiel Eudragit® RS), Copolymerisate aus Acryl- und Methacrylsäureestern und Trimethylammoniummethacrylat (zum Beispiel Eudragit® RL); Polyvinylacetat; Fette, Öle, Wachse, Fettalkohole; Hydroxypropylmethylcellulosephthalat oder -acetatsuccinat; Cellulose-acetatphthalat Stärke-acetatphthalat sowie Polyvinylacetatphthalat; Carboxymethylcellulose; Methylcellulosephthalat, Methylcellulosesuccinat, -phthalatsuccinat sowie Methylcellulose-phthalsäurehalbester; Zein; Ethylcellulose sowie Ethylcellulosesuccinat; Schellack, Gluten; Ethylcarboxyethylcellulose; Ethacrylat-Maleinsäureanhydrid-Copolymer; Maleinsäurenanhydrid-Vinylmethylether-Copolymer; Styrol-Maleinsäure-Copolymerisate: 2-Ethyl-hexyl-acrylatmaleinsäureanhydrid; Crotonsäure-Vinylacetat-Copolymer; Glutaminsäure/Glutaminsäureester-Copolymer; Carboxymethylethyl-celluloseglycerinmonooctanoat; Celluloseacetatsuccinat; Polyarginin.

Als Plastifizierungsmittel für Hüllstoffe können in Frage kommen:

Citronen- und Weinsäureester (Acetyltriethylcitrat, Acetyltributyl-, Tributyl-, Triethyl-citrat); Glycerin und Glycerinester (Glycerindiacetat, -triacetat, acetylierte Monoglyceride, Rizinusöl); Phthalsäureester (Dibutyl-, Diamyl-, Diethyl-, Dimethyl-, Dipropyl-phthalat), Di-(2-Methoxy- oder 2-ethoxyethyl)-phthalat), Ethylphthalylglycolat, Butylphthalylethylglycolat und Butylglycolat; Alkohole (Propylenglycol, Polyethylenglycol verschiedener Kettenlängen), Adipate (Diethyl -adipat, Di-(2-Methoxy-oder 2-ethoxyethyl)-adipat); Benzophenon; Diethyl- und Dibutylsebacat, Dibutylsuccinat, Dibutyltartrat; Diethylenglycoldipropionat; Ethylenglykoldiacetat, -dibutyrat, -dipropionat; Tributylphosphat, Tributyrin; Polyethylenglykolsorbitanmonooleat (Polysorbate wie Polysorbat 80); Sorbitanmonooleat.

Zur Herstellung von Lösungen oder Suspensionen kommen beispielsweise Wasser oder physiologisch verträgliche organische Lösungsmittel in Frage, wie zum Beispiel Ethanol, Propanol, Isopropanol, 1,2-Propylenglykol, Polyglykole und deren Derivate, Dimethylsulfoxid, Fettalkohole, Triglyceride, Partialester des Glycerins, Paraffine und ähnliche. Als Polyethylenglykole kommen zum Beispiel solche mit Molgewichten zwischen 100 und 6000, vorzugsweise 200 bis 4000 in Frage.

Für injizierbare Lösungen oder Suspensionen kommen zum Beispiel nicht-toxische parenteral verträgliche Verdünnungsmittel oder Lösungsmittel in Frage, wie zum Beispiel: Wasser, 1,3-Butandiol, Ethanol, 1,2-Propylenglykol, Polyglykole in Mischung mit Wasser, Glycerol, Ringer's Lösung, isotonische Kochsalzlösung, natürliche Öle (zum Beispiel Erdnußöl, Olivenöl, Sesamöl, Mandelöl, Sonnenblumenöl), Sojabohnenöl, Rizinusöl , Rinderfußöl) oder auch gehärtete Öle einschließlich synthetischer Mono-oder Diglyceride oder Fettsäuren wie Oleinsäure.

Bei der Herstellung der Zübereitungen können bekannte und übliche Lösungsvermittler, beziehungsweise Emulgatoren, verwendet werden. Als Lösungsvermittler und Emulgatoren kommen beispielsweise in Frage: Nikotinsäureamid, Polyvinylpyrrolidon, Sorbitanfettsäureester wie Sorbitantrioleat, Phosphatide, wie Lecithin, Acacia, Traganth, polyoxyethyliertes Sorbitanmonooleat und andere ethoxylierte Fettsäureester des Sorbitan, polyoxyethylierte Fette, polyoxyethylierte Oleotriglyceride, linolisierte Oleotriglyceride, Polyethylenoxyd-Kondensationsprodukte von Fettalkoholen, Alkylphenolen oder Fettsäuren oder auch 1-Methyl-3-(2-hydroxyethyl)imidazolidon-(2). Polyoxyethyliert bedeutet hierbei, daß die betreffenden Stoffe Polyoxyethylenketten enthalten, deren Polymerisationsgrad im allgemeinen zwischen 2 bis 40 und insbesondere zwischen 10 bis 20 liegt. Solche polyoxyethylierten Stoffe können beispielsweise durch Umsetzung von hydroxylgruppenhaltigen Verbindungen (beispielsweise Mono- oder Diglyceride oder ungesättigte Verbindungen wie zum Beispiel solchen, die Ölsäurereste enthalten) mit Ethylenoxyd erhalten werden (zum Beispiel 40 Mol Ethylenoxyd pro 1 Mol Glycerid).

Beispiele für Oleotriglyceride sind Olivenöl, Erdnußöl, Rizinusöl, Sesamöl, Baumwollsaatöl, Maisöl.

Siehe auch Dr. H. P. Fiedler "Lexikon der Hilfsstoffe für Pharmazie, Kosmetik und angrenzende Gebiete" 1971, S. 191-195.

Darüberhinaus ist der Zusatz von Konservierungsmitteln, Stabilisatoren, Puffersubstanzen, Geschmackskorrigentien, Süßmitteln, Farbstoffen, Antioxydantien und Komplexbildnern und dergleichen möglich.

Als Komplexbildner kommen beispielsweise in Frage: Chelatbildner wie Ethylendiamino-tetraessigsäure, Nitrilotriessigsäure, Diethylentriaminpentaessigsäure sowie deren Salze.

Weiterhin kommen als Komplexbildner auch solche in Frage, die Dihydroliponsäure in einem Hohlraum einschließen. Beispiele hierfür sind Harnstoff, Thioharnstoff, Cyclodextrine, Amylose.

Gegebenenfalls ist zur Stabilisierung des Wirkstoffmoleküls mit physiologisch verträglichen Basen oder Puffern auf einen pH-Bereich von ca. 6 bis 9 einzustellen. Im allgemeinen wird ein möglichst neutral er bis schwach basischer (bis pH 8) pH-Wert bevorzugt.

Als Antioxydantien kommen beispielsweise Natriumsulfit, Natriumhydrogensulfit, Natriummetabisulfit, Ascorbinsäure, Ascorbylpalmitat, -myristat, -stearat, Gallussäure, Gallussäure-alkylester, Butylhydroxyanisol, Nordihydroguajaretsäure, Tocopherole sowie Synergisten (Stoffe, die Schwermetalle durch Komplexbildung binden, beispielsweise Lecithin, Ascorbinsäure, Phosphorsäure Ethylendiaminotetraessigsäure, Citrate, Tartrate) zur Anwendung. Der Zusatz der Synergisten steigert die antioxygene Wirkung der Antioxydantien erheblich. Als Konservierungsmittel kommen beispielsweise Sorbinsäure, p-Hydroxybenzoesäureester (zum Beispiel Niederalkylester), Benzoesäure, Natriumbenzoat, Trichlorisobutylalkohol, Phenol, Kresol, Benzethoniumchlorid, Chlorhexidin und Formalinderivate in Betracht.

Die pharmazeutische und galenische Handhabung der Dihydroliponsäure erfolgt nach den üblichen Standardmethoden. Beispielsweise wird die Dihydroliponsäure und Hilfs- beziehungsweise Trägerstoffe durch Rühren oder Homogenisieren (zum Beispiel mittels üblicher Mischgeräte) gut vermischt, wobei im allgemeinen bei Temperaturen zwischen 20 und 80°C, vorzugsweise 20 bis 50° C, insbesondere bei Raumtemperatur gearbeitet wird. Im übrigen wird auf das folgende Standardwerk verwiesen: Sucker, Fuchs, Speiser, Pharmazeutische Technologie, Thieme-Verlag Stuttgart, 1978.

Die Applikation der Dihydroliponsäure beziehungsweise der Arzneimittel kann auf die Haut oder Schleimhaut oder in das Körperinnere erfolgen, beispielsweise oral, enteral, pulmonal, rectal, nasal, vaginal, lingual, intravenös, intraarteriell, intrakardial, intramuskulär, intraperitoneal, intracutan, subcutan.

Bei den parenteralen Zubereitungsformen handelt es sich insbesondere um sterile beziehungsweise sterilisierte Erzeugnisse.

Die Dihydroliponsäure kann auch in Form ihrer Salze verwendet beziehungsweise eingesetzt werden, wobei der Salzbildner auch im Überschuß eingesetzt werden kann, das heißt in einer höheren Menge als äquimolar.

Als Salzbildner für die Dihydroliponsäure kommen zum Beispiel übliche Basen oder Kationen in Frage, die in der Salzform physiologisch verträglich sind. Beispiele hierfür sind: Verträgliche Alkali- oder Erdalkalimetalle, Ammoniumhydroxid, basische Aminosäuren wie Arginin und Lysin, Amine der Formel $NR_1R_2R_3$ worin die Reste $R_1$, $R_2$ und $R_3$ gleich oder verschieden sind und Wasserstoff, $C_1$-$C_4$-Alkyl oder $C_1$-$C_4$ Oxyalkyl bedeuten wie Mono- und Diethanolamin, 1-Amino-2-propanol, 3-Amino-1-propanol; Alkylendiamine mit einer Alkylenkette aus 2 bis 6-C-Atomen wie Ethylendiamin oder Hexamethylentetramin, gesättigte cyclische Aminoverbindungen mit 4 - 6 Ringkohlenstoffatomen wie Piperidin, Piperazin, Pyrrolidin, Morpholin; N-Methylglucamin, Kreatin, Trometamol.

Kurze Beschreibung der in der Anmeldung besonders erwähnten pharmakologischen Testmethoden

Randall-Selitto-Test (Entzündungsschmerz an der Ratte):

In Anlehnung an die Methode von RANDALL und SELITTO (L.O. Randall u. J. Selitto, Arch. int. Pharmacodyn., Bd. 111, Seiten 409-418 (1957)) erhalten Ratten 0,1 ml einer 20%igen (in entmineralisiertem Wasser) Brauerhefesuspension in die rechte Hinterpfote subplantar injiziert. 2 1/2 Stunden danach werden die Prüfsubstanzen verabreicht und 30 Minuten später die Schmerzschwelle mit einem Algesiemeter als Druck (in Gramm) auf die entzündete Pfote gemessen (Geräte von Ugo BASILE, Mailand/Italien). Als Kriterium gilt die Abwehrreaktion der Tiere, die Pfote wegzuziehen und/oder sich aus dem Griff des Experimentators zu befreien. Die Substanzwirkung wird aus der Erhöhung der Schmerzschwelle gegenüber einer unbehandelten Kontrollgruppe ermittelt. Der Versuchsablauf unterscheidet sich von der Originalmethode dadurch, daß die Substanzen erst 2 1/2 Stunden nach der Ödemsetzung und nicht mit dieser gleichzeitig verabreicht werden. Auf diese Weise soll verhindert werden, daß die Ödementwicklung durch eine mögliche antiphlogistische Wirkung gehemmt und die Analgesie überlagert beziehungsweise vorgetäuscht wird.

Bestimmt wird die ED50 mittels der Methode der linearen Regression. Die ED50 ist hierbei die Dosis in mg/kg, bei der rechnerisch eine 50%ige analgetische Wirkung vorliegt.

Essigsäure-Test (Writhing-Test) an der Maus:

Methode:

Im Essigsäure-Test nach KOSTER et al. (Fed. Proc., Bd. 18, Seite 412 (1959)) wird der Schmerzreiz durch eine intraperitoneale Injektion 1%iger Essigsäure ausgelöst. Die Schmerzreaktion äußert sich als charakteristisches Strecken der Tiere ("writhing syndrome"), das in unregelmäßigen Zeitabständen längere Zeit nach der Essigsäure-Injektion anhält. Die dosisabhängige Hemmung der Häufigkeit der Streckbewegung gegenüber einer unbehandelten Kontrollgruppe wird als analgetische Wirkung in Prozent ausgedrückt. Die Auswertung erfolgt durch Bestimmung der ED50 (Methode der linearen Regression). Die ED50 ist die Dosis in mg/kg, bei der eine 50%ige Hemmung der "writhing syndrome" vorliegt.

Der Essigsäure-Test zeichnet sich dadurch aus, daß nicht nur die Wirkung von starken, zentral wirksamen Analgetika, sondern auch von überwiegend peripher wirksamen Analgetika-Antipyretika und antiphlogistisch wirksamen Pharmaka, wie Phenylbutazon, Indometacin u. a., nachweisbar ist. Damit weist die Wirkung in dieser Versuchsanordnung auf eine periphere Komponente der Analgesie hin.

Carrageenin-Ödem-Test auf antiphlogistische Wirkung:

Die Untersuchung erfolgt am Carrageenin-Ödem der Rattenpfote in Anlehnung an die Methode von MÖRSDORF und Mitarbeiter (Arch.int.Pharmacodyn. 192, 111-127 (1971)). Die antiphlogistische Wirkung wird z. B. als Ödemhemmung in Prozent gegenüber der unbehandelten Kontrollgruppe angegeben. Es wird bei sämtlichen Versuchen oral appliziert. Die Substanz wird 1 Stunde nach Auslösung der Entzündung oral beziehungsweise intraperitoneal verabreicht. Die ED50 ist die Dosis in mg/kg, bei der eine 50%ige Hemmung des Pfotenödems vorliegt.

Untersuchungen im PAF (Platelet-Aggregating-Factor)-Acether-Ödem an der Ratte

Prinzip der Methode ist es, durch subplantare Injektion von PAF-Acether 2 ug/Tier ein lokal begrenztes Ödem beziehungsweise eine Entzündung an der Rattenpfote zu erzeugen. Die Untersuchungen wurden in Anlehnung an die Methode nach SWINGLE und REITER (Agents and actions, 18, 358-365, (1986)) durchgeführt. Die Tiere erhielten die Prüfsubstanzen 0.5 Stunden vor der Auslösung des Ödems verabreicht. 1 Stunde danach wurde das Ödem ermittelt. Die ED50 wurde mittels der Methode der linearen Regression berechnet.

Beispiel 1

Ampullen mit 250 mg Dihydroliponsäure in 10 ml injektionslösung

60 g Trometamol und 1 g Ethylendiamintetraessigsäure, Dinatriumsalz werden in 1,8 Liter Wasser für Injektionszwecke gelöst. Die Lösung wird 30 Minuten lang mit Stickstoff begast. Unter weiterer Begasung mit Stickstoff werden in der Mischung 2 g Natriumdisulfit und anschließend 50 g Dihydroliponsäure gelöst. Die Lösung wird mit Wasser für Injektionszwecke, das mit Stickstoff begast wurde, auf ein Volumen von 2 Liter aufgefüllt. Nach sorgfältigem Mischen wird die Lösung über ein Membranfilter der Porenweite 0,2 μm filtriert und das Filtrat unter aseptischen Bedingungen und unter Vor- und Nachbegasung mit Stickstoff in Ampullen zu 10 ml Füllvolumen abgefüllt.

Eine Ampulle enthält in 10 ml Lösung 250 mg Dihydroliponsäure als Trometamolsalz.

Beispiel 2:

Suppositorien mit 200 mg Dihydroliponsäure

5 g Ascorbylpalmitat und 5 g Oxynex LM **) (E. Merck, Darmstadt) werden in 180 g geschmolzenem Hartfett *) suspendiert. Anschließend werden 20 g Dihydroliponsäure zugemischt und die Mischung nach Homogenisierung in Hohlzellen zu 2,3 ml ausgegossen und abgekühlt. Vor dem Verschließen werden die Hohlzellen mit Stickstoff begast.

Ein Suppositorium vom Gewicht 2,1 g enthält 200 mg Dihydroliponsäure.

Beispiel 3:

Creme mit 10 % Dihydroliponsäure

50 g Polyoxyethylen-40-stearat (Handelsname: Myrj®52), 80 g Cetylstearylalkohol, 200 g weißes Vaselin, 50 g dickflüssiges Paraffin und 5 g Dimethicone werden in einer Homogenisierungsapparatur zusammengeschmolzen. In der Schmelze werden 1,26 g Methyl-4-hydroxybenzoat und 0,533 g Propyl-4-hydroxybenzoat gelöst.

*) Hartfett ist ein Gemisch von Mono-, Di- und Triglyceriden der gesättigten Fettsäuren von $C_{10}H_{20}O_2$ bis $C_{18}H_{36}O_2$

**) Oxynex LM ist ein handelsüblicher Zusatzstoff für Fette und fetthaltige Lebensmittel. Er stellt eine hellbraune bis braune, wachsartige Masse dar, die beim Erwärmen auf 55 °C zu einer klaren braunen Flüssigkeit schmilzt und enthält -Tocopherol, Ascorbylpalmitat, Citronensäure und Lecithin.

EP 0 382 066 A2

In 511,207 g gereinigtem Wasser werden 1,4 g Methyl-4-hydroxybenzoat und 0,6 g Propyl-4-hydroxy-benzoat bei 70°C gelöst. Die Lösung wird in die oben erhaltene Fettschmelze einemulgiert. Die Emulsion wird homogenisiert und unter Rühren auf Raumtemperatur abgekühlt. Dann werden 100 g Dihydroliponsäure in die Creme eingerührt und unter Vakuum die Creme nochmals homogenisiert.

**Ansprüche**

1. Arzneimittel enthaltend als Wirkstoff Dihydroliponsäure oder ein pharmazeutisch verwendbares Salz der Dihydroliponsäure.

2. Arzneimittel enthaltend als Wirkstoff Dihydroliponsäure oder ein pharmazeutisch verwendbares Salz der Dihydroliponsäure sowie gegebenenfalls ein pharmazeutisch verträgliches Antioxydans und/oder einen Komplexbildner.

3. Arzneimittel nach einem oder mehreren der vorangegangenen Ansprüche, dadurch gekennzeichnet, daß es Dihydroliponsäure zusammen mit üblichen pharmazeutischen Trägern, Hilfsstoffen und/oder Verdünnungsmitteln enthält.

4. Arzneimittel nach einem oder mehreren der vorangegangenen Ansprüche, dadurch gekennzeichnet, daß die Dihydroliponsäure in einer Menge von 0,1 bis 600 mg vorliegt.

5. Arzneimittel nach einem oder mehreren der vorangegangenen Ansprüche, dadurch gekennzeichnet, daß es eine zytoprotektive, analgetische und/oder antiphlogistische Wirkung hat.

6. Verfahren zur Herstellung eines Arzneimittels, dadurch gekennzeichnet, daß Dihydroliponsäure oder ein pharmazeutisch verwendbares Salz der Dihydroliponsäure mit gebräuchlichen pharmazeutischen Trägerstoffen und/oder Verdünnungsmitteln beziehungsweise sonstigen Hilfsstoffen zu pharmazeutischen Zübereitungen verarbeitet beziehungsweise in eine therapeutisch anwendbare Form gebracht wird.

7. Verfahren zur Herstellung eines Arzneimittels, dadurch gekennzeichnet, daß man mindestens Dihydroliponsäure oder ein pharmazeutisch verwendbares Salz der Dihydroliponsäure zusammen mit üblichen Träger-und/oder Verdünnungs- beziehungsweise Hilfsstoffen bei Temperaturen zwischen 0 und 120°C, vorzugsweise 20 und 80°C vermischt beziehungsweise homogenisiert und gegebenenfalls die so erhaltene Mischung zur Herstellung von Zubereitungen, die in der Dosierungseinheit 0,1 bis 600 mg Dihydroliponsäure oder ein pharmazeutisch verwendbares Salz der Dihydroliponsäure enthalten, in Hohlzellen entsprechender Größe ausgießt oder in Kapseln entsprechender Größe abfüllt oder granuliert und dann gegebenenfalls unter Zusatz von weiteren üblichen Hilfsstoffen zu Tabletten verpreßt, oder in Kapseln abfüllt.

8. Verfahren zur Herstellung eines Arzneimittels, dadurch gekennzeichnet, daß man Dihydroliponsäure oder ein pharmazeutisch verwendbares Salz der Dihydroliponsäure gegebenenfalls mit einem Antioxydans sowie gegebenenfalls einem oder mehreren der folgenden Stoffe: Stärke, Cyclodextrin, Harnstoff, Cellulose, Lactose, Formalin-Casein, modifizierte Stärke, Magnesiumstearat, Calciumhydrogenphosphat, Kieselsäure, Talkum vermischt, die erhaltene Mischung gegebenenfalls mit einer wässrigen Lösung, die als Bestandteil mindestens Gelatine, Stärke, Polyvinylpyrrolidon, Vinyl pyrrolidon-Vinylacetat-Copolymerisat und/oder Polyoxyethylensorbitanmonooleat enthält, granuliert, das Granulat gegebenenfalls mit einem oder mehreren der oben genannten Hilfsstoffe homogenisiert, und diese Mischung zu Tabletten verpreßt oder in Kapseln abfüllt, wobei die Tabletten oder Kapseln in der Dosierungseinheit jeweils 0,1 bis 600 mg Wirkstoff Dihydroliponsäure oder ein Salz hiervon enthalten.

9. Verfahren zur Herstellung eines Arzneimittels, dadurch gekennzeichnet, daß man Dihydroliponsäure oder ein pharmazeutisch verwendbares Salz der Dihydroliponsäure gegebenenfalls mit 0,001 bis 1 Gewichtsteilen Antioxydans (bezogen auf 1 Gewichtsteil Dihydroliponsäure) sowie einem oder mehreren der folgenden Hilfsstoffe: Sojalecithin, Oxynex, bei Temperaturen zwischen 31 und 65°C in geschmolzenem Hartfett oder anderen, Fettsäureglyceride enthaltenden Mischungen mischt oder suspendiert und homogenisiert, und anschließend die Mischung in Hohlzellen ausgießt oder in Kapseln abfüllt, wobei die Dosierungseinheit 10 bis 600 mg Wirkstoff Dihydroliponsäure oder ein Salz hiervon enthält.

10. Verfahren zur Herstellung eines Arzneimittels, dadurch gekennzeichnet, daß man Dihydroliponsäure oder ein pharmazeutisch verwendbares Salz der Dihydroliponsäure, gegebenenfalls mit 0,001 bis 1 Gewichtsteilen (bezogen auf 1 Gewichtsteil Dihydroliponsäure) Antioxydans bei einer Temperatur zwischen 20 bis 120°C sowie gegebenenfalls in Gegenwart eines oder mehrerer Emulgatoren und/oder Komplexbildnern mit mindestens einem der folgenden Stoffe zu einer Mischung, die 0,5 bis 20 Gewichtsprozent Dihydroliponsäure enthält, homogenisiert und gegebenenfalls emulgiert: Wasser, Glycerin, Paraffin, Vaseline, aliphatischer Alkohol mit 12 bis 25 C-Atomen, aliphatische Monocarbonsäure mit 15 bis 20 C-Atomen, Sorbitanmonopalmitat, Polyoxyethylenpolyolfettsäureester, ein- oder mehrwertiger niedrigmolekularer aliphatischer Alkohol, Fettsäureglycerid, Wachs, Silikon, Polyethylenglykol.

9

11. Verfahren zur Herstellung eines Arzneimittels, dadurch gekennzeichnet, daß man Dihydroliponsäure oder ein pharmazeutisch verwendbares Salz der Dihydroliponsäure gegebenenfalls mit 0,001 bis 1 Gewichtsteilen (bezogen auf 1 Gewichtsteil Dihydroliponsäure) Antioxydans sowie gegebenenfalls in Anwesenheit eines Komplexbildners und/oder eines Emulgators, bei Temperaturen zwischen 30 und 100°C in Wasser, physiologisch unbedenklichen Alkoholen, Dimethylsulfoxid, Polyethylenglykol oder Ölen oder Mischungen hiervon auflöst, und gegebenenfalls die so erhaltene Lösung mit soviel Wasser, Alkohol, Dimethylsulfoxid, Polyethylenglykol oder Öl auffüllt, daß die Endlösung, Endsuspension oder Endemulsion 0,5 - 20 Gewichtsprozent an Wirkstoff Dihydroliponsäure enthält.

12. Verwendung von Dihydroliponsäure und deren pharmazeutisch verwendbare Salze zur Herstellung von Arzneimitteln mit zytoprotektiver Wirkung sowie zur Bekämpfung von Schmerz- und Entzündungserkrankungen.

13. Verwendung von Dihydroliponsäure und deren pharmazeutisch verwendbaren Salzen gemäß Anspruch 12 dadurch gekennzeichnet, daß Arzneimittel mit zytoprotektiver Wirkung sowie zur Bekämpfung von Schmerz-und Entzündungserkrankungen hergestellt werden.